(19) 

Europäisches
Patentamt
European
Patent Office
Office européen
des brevets

(11) **EP 4 268 870 B1**

(12) **EUROPEAN PATENT SPECIFICATION**

(45) Date of publication and mention
of the grant of the patent:
**11.03.2026 Bulletin 2026/11**

(21) Application number: **21919802.5**

(22) Date of filing: **15.04.2021**

(51) International Patent Classification (IPC):
***A61M 16/00*** *(2006.01)*    ***A61M 16/10*** *(2006.01)*
***A61B 5/08*** *(2006.01)*

(52) Cooperative Patent Classification (CPC):
**A61B 5/087; A61M 16/0069; A61M 16/024;
A61M 16/109; A61M 16/16;** A61M 16/0672;
A61M 2016/0021; A61M 2016/0027;
A61M 2016/0039; A61M 2205/3331;
A61M 2205/3334; A61M 2205/3365

(86) International application number:
**PCT/KR2021/004770**

(87) International publication number:
**WO 2022/154175 (21.07.2022 Gazette 2022/29)**

(54) **HIGH FLOW RESPIRATORY THERAPY DEVICE THROUGH BREATH SYNCHRONIZATION**

ATEMTHERAPIEVORRICHTUNG MIT HOHEM DURCHFLUSS DURCH ATEMSYNCHRONISATION

DISPOSITIF DE THÉRAPIE RESPIRATOIRE À HAUT DÉBIT PAR SYNCHRONISATION
RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.01.2021 KR 20210004258**

(43) Date of publication of application:
**01.11.2023 Bulletin 2023/44**

(73) Proprietor: **Mek Co., Ltd.
Paju-si, Gyeonggi-do 10911 (KR)**

(72) Inventor: **KIM, Jong Cheol
Goyang-si Gyeonggi-do 10395 (KR)**

(74) Representative: **Gallo, Luca
Gallo & Partners S.r.l.
Via Rezzonico, 6
35131 Padova (IT)**

(56) References cited:
JP-B2- 6 058 676    KR-A- 20160 098 918
KR-A- 20190 001 332    KR-A- 20190 010 874
US-A1- 2009 301 487    US-A1- 2017 326 315
US-A1- 2019 217 030    US-A1- 2020 405 984

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

EP 4 268 870 B1

## Description

### Technical Field

The present invention relates to a high flow respiratory therapy device, and more specifically, to high flow respiratory therapy device through breath synchronization, which can detect the time point when a patient attempts to start inhalation and the time point when a patient attempts to start exhalation, reduce a dead space by supplying a high flow of mixed Gas during inhalation, and reduce the patient's respiratory effort by decreasing a flow rate of mixed gas during exhalation.

### Background Art

High flow respiratory therapy means therapy assisting a patient's respiration by relatively reducing a dead space where gas exchange does not occur by delivering air heated and humidified at concentration higher than the oxygen concentration in the atmosphere, at a flow rate two to three times or more than the patient's breathing volume.

In general, most of the air goes into the alveoli for gas exchange, and some remains in the respiratory tract (or airway). The volume of air that stays in the airway is called the dead space, where no gas exchange takes place during actual respiration.

For instance, in a case in which the volume of the dead space is about 150 ml when the tidal volume of a healthy person is 500 ml, the actual tidal volume becomes 350 ml. However, a patient with decreased respiratory capacity, whose tidal volume is only 300 ml, would have an actual breathing volume of 150 ml after excluding the dead space of 150 ml, which is less than half the normal level. Therefore, if the patient's tidal volume is not sufficiently larger than the dead space, the patient may experience respiratory failure, namely, respiratory-deficient.

Therefore, the patient may breathe faster or consume a lot of energy to maintain normal O2 saturation (SaO2 or SpO2), and it may deteriorate the patient's condition.

To solve the above problem, conventionally, a method of providing a constant flow of mixed gas to a patient using a high flow respiratory therapy device have been used. The administration method may include using a nasal cannula, which is a thin tube inserted into both nostrils and hung over the ears, or an oxygen mask.

However, the conventional high flow respiratory therapy device can reduce the patient's effort to breathe during inhalation because a supply flow to the patient is greater than an inhalation flow by the patient's spontaneous respiration. However, the conventional high flow respiratory therapy device may give burden to the patient's expiratory effort since air resistance increases compared to the spontaneous respiration due to high flow nasal delivery during exhalation.

Additionally, the start of the patient's inhalation entirely depends on the patient's will to breathe. In contrast, the conventional high flow respiratory therapy device has an effect of the high flow therapy since delivering mixed gas at the same flow rate regardless of inhalation and exhalation time points, which may vary according to the patient's condition, but cannot reduce the burden on the patient's inspiratory effort or expiratory effort.

Especially, the high flow respiratory therapy using a nasal cannula diversely detects resistance, volume, and breathing effort according to the size of the patient's nostrils, the state of cannula mounting, and the anatomical characteristics of the nasal cavity and upper airway even though mixed gas of the same flow rate is supplied, so it is difficult to perform active respiratory therapy to reduce the expiratory effort due to technical difficulty in synchronized detection (detection at the time of inhalation/exhalation).

Document US 2019/217030 A1 discloses a flow therapy apparatus provided with flow sensors to detect the patient's respiratory cycle and control the gas flow provided to the patient based on the detected respiratory cycle. In particular, the apparatus detects a patient breath cycle on the basis of several measurements, such as a flow rate measurement. Furthermore, the apparatus determines if the patient is currently inhaling or exhaling. If the patient is inhaling, the apparatus increases air flow to the patient, and if the patient is exhaling, the apparatus decreases air flow to the patient. Specifically, the amount of increase or decrease in the airflow may be based upon a magnitude of inspiration/expiration by the patient.

### Disclosure

### Technical Problem

Accordingly, the present invention has been made in view of the above-mentioned problems occurring in the related art, and it is an object of the present invention to provide high flow respiratory therapy device through breath synchronization, which can detect the time point when a patient attempts to start inhalation and the time point when a patient attempts to start exhalation, reduce a dead space by supplying a high flow of mixed gas during inhalation, and reduce the patient's respiratory effort by decreasing a flow rate of mixed gas during exhalation.

## Technical Solution

To accomplish the above-mentioned objects, according to the present invention, there is provided a high flow respiratory therapy device through breath synchronization, as defined in claim 1. The device includes: a breathing pattern monitoring part, which monitors flow rate and pressure changes of a mixed gas supplied to a patient and collects the patient's breathing pattern information; an inspiratory effort point detection part, which detects an inspiratory effort point, at which the patient tries to start inhalation, from the patient's breathing pattern information; an expiratory effort point detection part, which detects a expiratory effort point, at which the patient tries to start exhalation, from the patient's breathing pattern information; and a supply flow control part, which increases the flow of the mixed gas when the patient's respiration reaches the inspiratory effort point and decreases the flow of the mixed gas when the patient's respiration reaches the expiratory effort point.

Moreover, the high flow respiratory therapy device further includes a breathing synchronization unit, which includes the breathing pattern monitoring part, the inspiratory effort point detection part, and the expiratory effort point detection part, and synchronizes the extracted inspiratory effort point and the expiratory effort point with the patient's breathing pattern information, wherein the supply flow control part synchronizes the flow control of the mixed gas with respect to the inspiratory effort point and expiratory effort point synchronized in the breathing synchronization unit.

## Advantageous Effects

The present invention supplies a high flow of a mixed gas sufficient to ventilate the patient's nasal cavity with fresh air during inhalation, thereby clinically reducing the dead space, and reducing the patient's respiratory effort by reducing the patient's expiratory effort.

## Description of Drawings

FIGS. 1 and 2 are diagrams illustrating the configuration of a high flow respiratory therapy device according to an embodiment of the present invention.

FIG. 3 is a diagram illustrating the configuration of the high flow respiratory therapy device according to a first embodiment of the present invention in the device of FIGS. 1 and 2.

FIG. 4 is a graph showing a value that changes in flow rate of a mixed gas are monitored according to the first embodiment of the present invention.

FIGS. 5 and 6 are waveform diagrams illustrating an example of extracting an inspiratory effort point and an expiratory effort point of a patient according to the patient's respiratory changes according to the first embodiment of the present invention.

FIG. 7 is an operation flow chart for depicting a high flow respiratory therapy method through breathing synchronization, not falling within the scope of the claims, actuated by the first embodiment of the present invention.

FIG. 8 is a diagram illustrating the configuration of a high flow respiratory therapy device according to a second embodiment, which does not fall within the scope of the claims.

FIG. 9 is a graph showing a value that changes in flow rate of a mixed gas are monitored according to the second embodiment.

FIG. 10 is a waveform diagram illustrating an example of extracting an inspiratory effort point and an expiratory effort point of a patient according to the patient's respiratory changes according to the second embodiment.

FIG. 11 is an operation flow chart for depicting a high flow respiratory therapy method through breathing synchronization actuated by the second embodiment.

## Mode for Invention

Hereinafter, embodiments of the present invention will be described in detail with reference to the accompanying drawings.

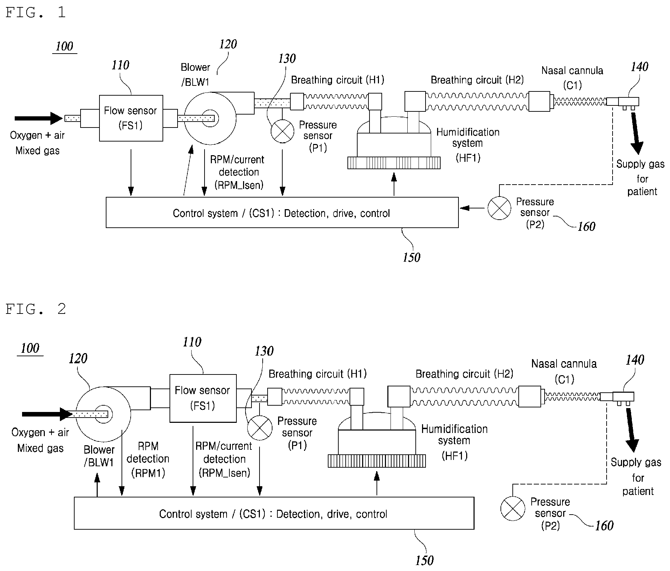

FIGS. 1 and 2 are diagrams illustrating the configuration of a high flow respiratory therapy device according to an embodiment of the present invention.

The high flow respiratory therapy device 100 according to an embodiment of the present invention includes a mixed gas generating unit, which generates mixed gas containing oxygen and air, a flow sensor 110 (in FIGS. 1 and 2 ), a blower 120 (in FIGS. 1 and 2 ), a pressure sensor 130 (in FIGS. 1 and 2 ), a humidifier HF1 (in FIGS. 1 and 2 ), a nasal cannula C1 (in FIGS. 1 and 2 ) which has a supply hose 140 (in FIGS. 1 and 2 ), and a control part 150 (in FIGS. 1 and 2 ).

The flow sensor FS1 may be installed at the front end (see FIG. 1) or the rear end (see FIG. 2) of the blower 120 depending on the position and configuration to be sensed.

The pressure sensor 130 may be additionally placed close to the patient like P2 of FIGS. 1 and 2. The pressure sensor 160 at the rear end can measure the flow rate and pressure supplied to the patient close to reality, but the measurement results may significantly vary depending on the mounted state of the nasal cannula 140 or the installation status of the cannula 140. By the above configuration, the high flow respiratory therapy device 100 administers a heated and humidified mixed gas with oxygen concentration higher than that of the atmosphere to the patient. In this instance, the high flow respiratory therapy device 100 adjusts the flow rate and flow volume of the mixed gas depending on the inhalation and exhalation points of the patient's breath.

In addition, in order to solve the problem that the resistance, volume, and respiratory effort are differently detected according to the patient's nostril size, the installation status of the cannula, and the anatomical characteristics of the nasal cavity and the upper airway, the high flow respiratory therapy device 100 according to an embodiment of the present invention detects the inhalation and exhalation points based on the pressure and the flow rate supplied to the patient and synchronizes them with the patient's respiratory cycle. For this, the control part 150 must precede the breathing synchronization process which monitors and synchronizes the patient's breath before treatment.

Therefore, the high flow respiratory therapy device 100 according to an embodiment of the present invention reduces the patient's respiratory effort by increasing or decreasing the flow rate of the mixed gas at each inhalation point or exhalation point, referring to the synchronized inhalation and exhalation points in the control part 150.

Specifically, two synchronization plans for the high flow respiratory therapy device 100 according to an embodiment of the present invention will be provided.

First Embodiment

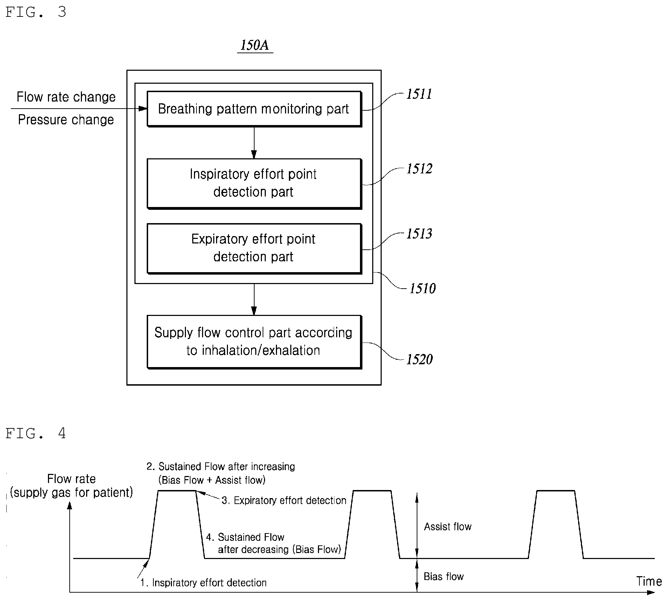

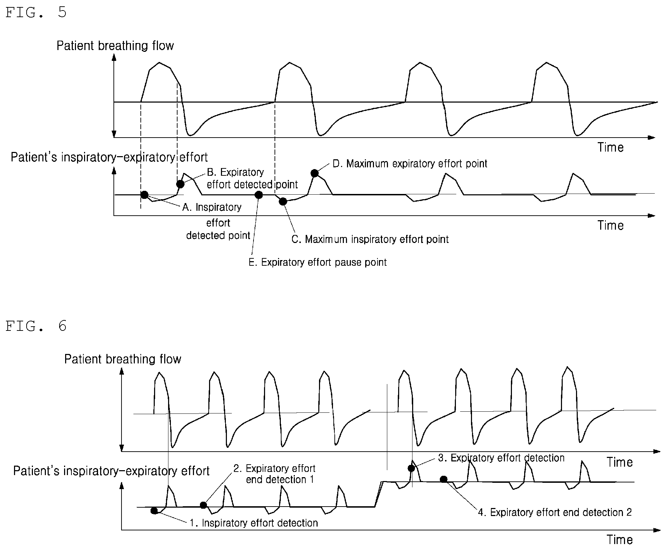

FIG. 3 is a diagram illustrating the configuration of the high flow respiratory therapy device according to a first embodiment of the present invention in the device of FIGS. 1 and 2 , FIG. 4 is a graph showing a value that changes in flow rate of a mixed gas are monitored according to the first embodiment of the present invention, and FIGS. 5 and 6 are waveform diagrams illustrating an example of extracting an inspiratory effort point and an expiratory effort point of a patient according to the patient's respiratory changes according to the first embodiment of the present invention.

For the sake of convenience in understanding, FIG. 3 will be described with reference to the waveforms illustrated in FIGS. 4 to 6.

The control part 150A of the high flow respiratory therapy device according to the first embodiment of the present invention includes: a breathing synchronization part 1510, which includes a breathing pattern monitoring part 1511, an inspiratory effort point detection part 1512, and an expiratory effort point detection part 1513; and a supply flow control part 1520 according to inhalation/exhalation.

Here, the supply flow control part 1520 according to inhalation/exhalation is configured to control a supply flow rate in conjunction with the breathing synchronization part 1510, and even if it is a control part which controls the flow rate with the same function, may be distinguished according to the functional role.

The breathing pattern monitoring part 1511 monitors the flow rate change and the pressure change of the mixed gas supplied to the patient using the flow sensor and pressure sensor of the high flow respiratory therapy device 100 (in FIGS. 1 and 2). Through such monitoring, the breathing pattern monitoring part collects the patient's breathing pattern information.

In addition, the breathing pattern information and the respiratory effort information of FIG. 5 may include flow rate and pressure information detected from the flow sensor and the pressure sensor. The patient respiration-related waveform illustrated in FIG. 5 shows that the patient's flow rate increases at the inspiratory effort point and decreases at the expiratory effort point.

The inspiratory effort point detection part 1512 detects the inspiratory effort point A (in FIG. 5), at which the patient tries to start inhaling, from the patient's breathing pattern information collected in the breathing pattern monitoring part 1511.

Simultaneously, the expiratory effort point detection part 1513 detects the expiratory effort point B (in FIG. 5), at which the patient tries to start exhaling, from the breathing pattern information collected in the breathing pattern monitoring part 1511.

Specifically, the inspiratory effort point detection part 1512 extracts a maximum inspiratory effort point C in (FIG. 5), at which the change in inhalation flow rate is maximized, and an expiratory effort end point E (in FIG. 5), at which respiration temporarily stops before inhaling again after exhaling, from the patient's breathing pattern information. Thereafter, the inspiratory effort point detection part detects an inspiratory effort point A (in FIG. 5) at which the patient starts (initiates) an inspiratory effort in between the extracted expiratory effort end point E (in FIG. 5) and the maximum inspiratory effort point C (in FIG. 5), with reference to the extracted expiratory effort end point E and the maximum inspiratory effort point C.

At this time, the inspiratory effort point detection part 1512 detects the expiratory effort end point from an average value obtained by adding the patient's respiratory flow rate during one cycle and dividing by the number of counts, and can extract where the maximum inspiratory effort point and the maximum expiratory effort point are located relative to the expiratory effort end point as the zero point.

In addition, the inspiratory effort point detection part 1512 calculates a scalar value between the expiratory effort end point E (in FIG. 5) in the first cycle and the maximum inspiratory effort point C (in FIG. 5) in the second cycle, referring to at least two cycles, and detect a value within a certain range of 10% to 30% from the maximum inspiratory effort point C (in FIG. 5) based on the calculated scalar value as the inspiratory effort point A (in FIG. 5). Alternatively, not according to the invention, the inspiratory effort point detection part 1512 can calculate a gradient between the expiratory effort end point E (in FIG. 5) in the first cycle and the maximum inspiratory effort point C (in FIG. 5) in the second cycle, and detect the inspiratory effort point A (in FIG. 5) based on the calculated gradient.

The range of 10% to 30% can be set differently according to the patient's condition, the mounted state of the cannula, and so on.

The effort points and the end points are calculated in relation to the following equation.

$$\text{Effort} = \text{Pressure} \times \text{Volume} / \text{time} = \text{Pressure} \times \text{Flow (L / min)}$$

The expiratory effort point detection part 1513 extracts the maximum expiratory effort point D (in FIG. 5), at which the change in the patient's exhalation flow rate is maximized, and an expiratory effort endpoint E (in FIG. 5), at which respiration temporarily stops before inhaling again after exhaling, from the patient's breathing pattern information. Thereafter, the expiratory effort point detection part detects an expiratory effort point B (in FIG. 5) at which the patient starts (initiates) an expiratory effort in between the extracted expiratory effort end point E (in FIG. 5) and the maximum expiratory effort point D (in FIG. 5), with reference to the extracted expiratory effort end point E and the maximum expiratory effort point D.

The expiratory effort end point E (in FIG. 5) is extracted from an average value obtained by adding the patient's respiratory flow rate during one cycle and dividing by the number of counts.

The expiratory effort point detection part 1513 can extract where the maximum expiratory effort point is located relative to the expiratory effort end point E (in FIG. 5) as the zero point.

Furthermore, the expiratory effort point detection part 1513 calculates a scalar value between the expiratory effort end point E (in FIG. 5) in the first cycle and the maximum expiratory effort point D (in FIG. 5) in the second cycle, referring to at least two cycles, and detect a value within a certain range of 10% to 30% from the maximum expiratory effort point D (in FIG. 5) based on the calculated scalar value as the expiratory effort point. Alternatively, not according to the invention, the expiratory effort point detection part can calculate a gradient between the expiratory effort end point E (in FIG. 5) in the first cycle and the maximum expiratory effort point D (in FIG. 5) in the second cycle, and detect the expiratory effort point based on the calculated gradient.

The detected inspiratory effort point A and expiratory effort point B correspond to the time points before becoming the maximum inspiratory effort point C and the maximum expiratory effort point D respectively. Therefore, when the flow rates at the time points of the inspiratory effort point and the expiratory effort point are controlled and supplied, the patient's inspiratory effort or expiratory effort is effectively reduced at the time point when the patient inhales or exhales to the maximum.

The respiration synchronization part 1510 is configured to include the breathing pattern monitoring part 1511, the inspiratory effort point detection part 1512, and the expiratory effort point detection part 1513, and synchronizes the inspiratory effort point and the expiratory effort point extracted from the inspiratory effort point detection part 1512 and expiratory effort point detection part 1513 in the patient's breathing pattern information. Accordingly, the respiration synchronization part 1510 synchronizes the inhalation point and the exhalation point in the patient's respiratory cycle.

The supply flow rate control part 1520 synchronizes and performs the flow rate control of the mixed gas with respect to the inspiratory effort point and the expiratory effort point synchronized in the respiration synchronization part 1510.

That is, in the patient's breathing cycle, when reaching at the inspiratory effort point, the supply flow control part 1520 synchronizes at the point and increases the flow rate of the mixed gas, and when reaching the expiratory effort point, the supply flow control part decreases the flow rate of the mixed gas.

At this time, the supply flow control part 1520 sets a flow rate that can ventilate the patient's nasal cavity with fresh air as a basic flow (bias flow). The supply flow control part 1520 usually supplies air at the bias flow, but supplies air by adding a plus auxiliary flow (assist flow) to the bias flow at the time of inhalation.

The assist flow may vary according to the patient's condition, the patient's breath size, and the mounted state of the cannula.

The flow rate to fill the nasal cavity with fresh air can be determined based on clinical or anatomical criteria.

Therefore, in a case in which the patient's breathing starts or the operation of the cannula starts after the cannula is installed, the high flow respiratory therapy device according to the first embodiment of the present invention supplies the mixed gas of the bias flow through the control of the control part 150A, and supplies the inhalation flow rate which increased by the assist flow amount from the bias flow when reaching the inspiratory effort point. when the patient's inhalation ends and the expiratory effort point is detected after continuously supplying the inhalation flow rate, the high flow respiratory therapy device performs an operation to reduce the inhalation flow rate by switching to the bias flow.

Through the above operation, the supply flow rate like the waveform illustrated in FIG. 4 is obtained.

Meanwhile, the waveform illustrated in the lower portion of FIG. 5 is the waveform of the patient's breathing effort as a result of performing the respiration synchronization process based on the bias flow in the device according to the first embodiment of the present invention.

The baseline where the cycle starts in FIG. 5 corresponds to a value corresponding to the bias flow.

On the other hand, FIG. 6 illustrates the waveform of the patient's breathing effort as a result of performing the breathing synchronization process not only based on the bias flow but also the inhalation flow in the device according to the first embodiment of the present invention.

That is, the first half of the cycle is a waveform performed by the breathing synchronization process at the bias flow, and the latter half of the cycle is a waveform performed by the breathing synchronization process at the inhalation flow (bias flow + assist flow). It is confirmed that the baseline between the first half of the cycle and the latter half of the cycle has changed.

Such a synchronization method can be applied when the therapeutic flow rate changes, and may be a process of breathing synchronization to match a new flow rate.

Meanwhile, in the embodiment of FIG. 6, when detecting the inspiratory effort point and the expiratory effort point, with respect to the mixed gas flow rate of FIG. 4, the inspiratory effort point occurs when supplying the bias flow, and the expiratory effort point occurs when supplying the inhalation flow. Therefore, in a case of detecting each effort point, especially when detecting the inspiratory effort point, it is advantageous to synchronize the bias flow and detect it at the bias flow, and when detecting the expiratory effort point, it is advantageous to detect it at the inhalation flow. This is one example of detection methods and is not limited thereto, and it would be also possible to detect the effort points at the bias flow regardless of the flow rate.

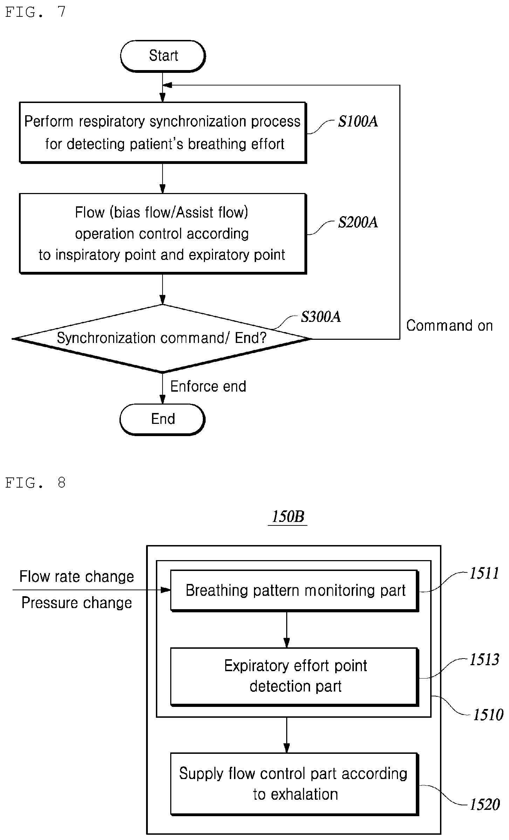

Meanwhile, FIG. 7 is an operation flow chart for depicting a high flow respiratory therapy method through breathing synchronization, not falling with the scope of the claims, actuated by the first embodiment of the present invention.

In step S100A, the control part 150A performs a breathing synchronization process to detect the patient's respiratory effort.

Specifically, the breathing pattern monitoring part 1511 in the control part 150A monitors changes in flow rate and pressure of the mixed gas supplied to the patient and collects information on the patient's breathing pattern.

Subsequently, the detection part of the control part 150A detects an inspiratory effort point at which the patient is trying to start inhaling, and an expiratory effort point at which the patient is trying to start exhaling, or only the expiratory effort point, from the patient's breathing pattern information collected in the previous process.

For example, the inspiratory effort point detection part 1512 of the control part 150A detects the inspiratory effort point, and the expiratory effort point detection part 1513 of the control part 150A detects the expiratory effort point.

Thereafter, the breathing synchronization unit 1510 of the control part 150A synchronizes the detected inspiratory effort point and expiratory effort point with the patient's breathing pattern information.

Generally, the start of the patient's inhalation depends on the patient's will to breathe. Through the breathing synchronization process, the high flow respiratory therapy device can synchronize the actual inhalation point and the actual exhalation point of the patient with the respiratory cycle, respectively.

In the step S200A, the supply flow control part 1520 of the control part 150A actively controls the flow rate of the mixed gas corresponding to the inspiratory effort point or the expiratory effort point, and especially, performs flow control in synchronization with the inspiratory effort point and the expiratory effort point, which are synchronized in the breathing synchronization unit.

Specifically, the supply flow control part 1520 supplies mixed gas of a predetermined bias flow when the patient's respiration starts, supplies the mixed gas by adding assist flow to the bias flow at the inspiratory effort point, and supplies the mixed gas by reducing to the bias flow at the expiratory effort point.

In the step S300A, if there is an additional command for synchronization by the manipulation of the user or the doctor, the control part 150A moves back to the step S100A and operates, and stops the operation when synchronization end is input.

Second Embodiment (not falling within the scope of the claims)

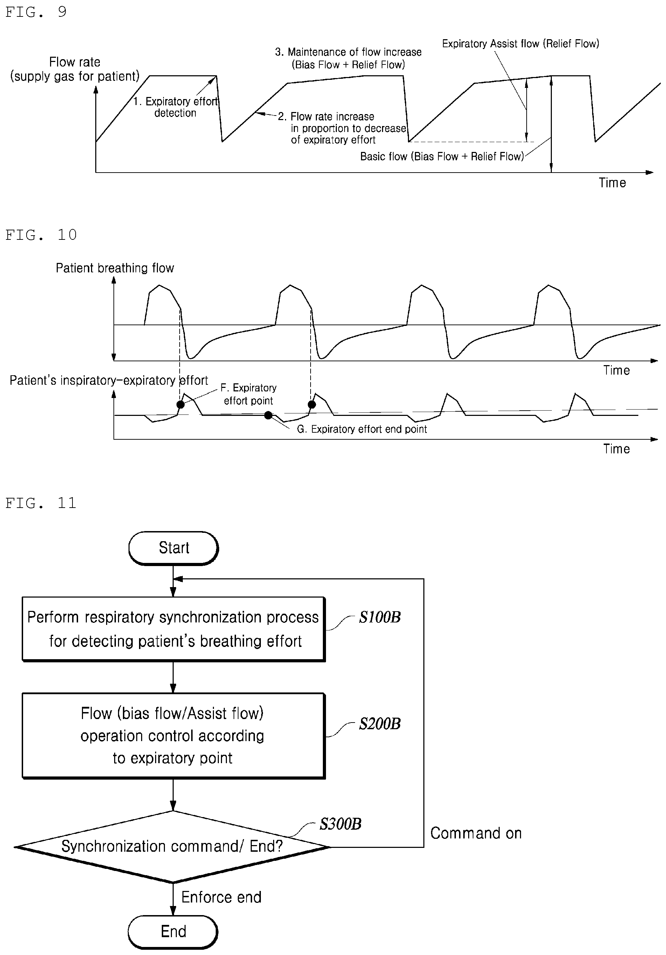

FIG. 8 is a diagram illustrating the configuration of a high flow respiratory therapy device according to a second embodiment (not falling within the scope of the claims), FIG. 9 is a graph showing a value that changes in flow rate of a mixed gas are monitored according to the second embodiment, and FIG. 10 is a waveform diagram illustrating an example of extracting an inspiratory effort point and an expiratory effort point of a patient according to the patient's respiratory changes according to the second embodiment.

Referring to FIG. 8, the high flow respiratory treatment device according to the second embodiment includes a control part 150B including a breathing synchronization unit 1510, which has a breathing pattern monitoring part 1511, and an expiratory effort point detection part 1513, and a supply flow control part 1520.

Each unit performs the same functions as the components according to the first embodiment. However, in the second embodiment, the supply flow control part 1520 of the second embodiment is different from that of the first embodiment in that the supply flow control part 1520 of the second embodiment detects only the expiratory effort point to control the flow rate of the mixed gas.

The breathing pattern monitoring part 1511 monitors at least one of the flow rate change and the pressure change of the mixed gas supplied to the patient, and collects information on the patient's breathing pattern.

The expiratory effort point detection part 1513 detects an expiratory effort point, at which the patient tries to start exhalation, from the breathing pattern information collected by the breathing pattern monitoring part 1511. The detection method is the same as described above.

The breathing synchronization unit 1510 includes the breathing pattern monitoring part 1511 and the expiratory effort point detection part 1513, and synchronizes an expiratory effort point from the expiratory effort point detection part 1513 with the patient's breathing pattern information. Through this, an exhalation point is synchronized with the patient's respiratory cycle.

The supply flow control part 1520 also synchronizes the control of the mixed gas flow with respect to the expiratory effort point synchronized in the breathing synchronization unit 1510.

In other words, when the patient's respiration starts, the supply flow control part 1520 supplies and maintains air at a predetermined bias flow, and reduces the flow rate of the mixed gas by an exhalation minus-auxiliary flow (relief flow) at an expiratory effort point and supplies the exhalation flow, thereby reducing the patient's expiratory effort. Thereafter, when the expiratory effort is reduced, as illustrated in FIG. 9, the supply flow control part 1520 increases the flow rate in proportion to the decrease of the expiratory effort. When the flow rate is increased to the bias flow, the supply flow control part 1520 maintains the bias flow till the next expiratory effort point is detected.

In the second embodiment, the bias flow is determined to include the flow level which can ventilate the patient's nasal cavity with fresh air and the patient's maximum inhalation flow.

The patient's maximum inhalation flow can be determined to be three to four times the breathing capacity per minute clinically. The breathing capacity per minute is calculated as [one breath volume] $\times$ [number of breaths per minute].

For example, assuming that one breathing capacity for adults is 500 ml and the average number of breaths per minute is 14, the breathing capacity per minute is approximately 7 L/min, and accordingly, the maximum inhalation flow is determined to be 20 to 30 LPM.

As described above, the flow level to fill the nasal cavity with fresh air and the patient's maximum inhalation flow can be determined based on clinical or anatomical criteria.

FIG. 10 shows the waveform for the patient's expiratory effort as a result of performing the breathing synchronization process of the device according to the second embodiment based on the bias flow.

Hereinafter, the operation of the high flow respiratory treatment device according to a second embodiment will now be described.

Referring to FIG. 11, in step S100B, the control part 150B performs a breathing synchronization process to detect the patient's respiratory effort.

Specifically, the breathing pattern monitoring part 1511 in the control part 150B monitors the flow and pressure changes of the mixed gas supplied to the patient, and collects information on the patient's breathing pattern.

Subsequently, the expiratory effort point detection part 1513 in the control part 150B detects only the expiratory effort points at which the patient tries to start exhalation from the collected patient's breathing pattern information through the above process.

Thereafter, the breathing synchronization unit 1510 in the control part 150B synchronizes the detected expiratory effort points with the patient's breathing pattern information.

In the next step S200B, the supply flow control part 1520 in the control part 150B actively controls the flow rate of the mixed gas corresponding to the expiratory effort point, in particular, can synchronize the flow control with the expiratory effort point synchronized in the breathing synchronization unit 1510.

Specifically, the supply flow control part 1520 supplies mixed gas of a predetermined bias flow when the patient's respiration starts, and then, and reduces the flow rate of the mixed gas by an exhalation minus auxiliary flow (relief flow) at an expiratory effort point and supplies the exhalation flow. Thereafter, the supply flow control part 1520 increases the flow rate in proportion to the decrease of the expiratory effort, and when reaching the bias flow, maintains the bias flow till the next expiratory effort is detected.

In step S300B, if there is an additional command for synchronization by the manipulation of the user or the doctor, the control part 150B moves back to the step S100B and operates, and stops the operation when synchronization end is input.

The scope of the invention is defined by the following claims.

## Claims

1. A high flow respiratory therapy device (100) through breath synchronization comprising:

   a breathing pattern monitoring part (1511), which is configured to monitor flow rate and pressure changes of a mixed gas supplied to a patient and is configured to collect the patient's breathing pattern information;
   an inspiratory effort point detection part (1512), which is configured to detect an inspiratory effort point (A), at which the patient tries to start inhalation, from the patient's breathing pattern information;
   an expiratory effort point detection part (1513), which is configured to detect an expiratory effort point (B), at which the patient tries to start exhalation, from the patient's breathing pattern information; and
   a supply flow control part (1520), which is configured to increase the flow of the mixed gas when the patient's respiration reaches the inspiratory effort point (A) and is configured to decrease the flow of the mixed gas when the patient's respiration reaches the expiratory effort point (B);
   **characterized in that**:

   the inspiratory effort point detection part (1512) is configured to extract a maximum inspiratory effort point (C), at which the change in inhalation flow rate is maximized, and an expiratory effort end point (E), at which respiration temporarily stops before inhaling again after exhaling, from the patient's breathing pattern information, and is configured to detect the inspiratory effort point (A) at which the patient starts an inspiratory effort between the extracted expiratory effort end point (E) and the maximum inspiratory effort point (C) referring to the extracted expiratory effort endpoint (E) and the maximum inspiratory effort point (C);
   the expiratory effort end point (E) is extracted from an average value obtained by adding the patient's respiratory flow rate during one cycle and dividing by the number of counts;
   the inspiratory effort point detection part (1512) is configured to calculate a scalar value between the expiratory effort end point (E) in a first cycle and the maximum inspiratory effort point (C) in a second cycle, referring to at least two cycles, and detect a value within a range of 10% to 30% from the maximum inspiratory effort point (C) based on the calculated scalar value as the inspiratory effort point (A);
   the expiratory effort point detection part (1513) is configured to extract a maximum expiratory effort point (D), at which the change in the patient's exhalation flow rate is maximized, and the expiratory effort end point (E), at which respiration temporarily stops before inhaling again after exhaling, from the patient's breathing pattern information, and is configured to detect the expiratory effort point (B) at which the patient starts an expiratory effort between the extracted maximum expiratory effort point (D) and the expiratory effort end point (E) referring to the extracted maximum expiratory effort point (D) and the expiratory effort end point (E);
   the expiratory effort point detection part (1513) is configured to calculate a scalar value between the expiratory effort end point (E) in a first cycle and the maximum expiratory effort point (D) in a second cycle, referring to at least two cycles, and detect a value within a range of 10% to 30% from the maximum expiratory effort point (D) based on the calculated scalar value as the expiratory effort point.

2. The high flow respiratory therapy device (100) according to claim 1, further comprising:

   a breathing synchronization unit (1510), which includes the breathing pattern monitoring part (1511), the inspiratory effort point detection part (1512), and the expiratory effort point detection part (1513), and is configured to synchronize the inspiratory effort point (A) extracted by the inspiratory effort point detection part (1512) and the expiratory effort point (B) extracted by the expiratory effort point detection part (1513) with the patient's breathing pattern information,
   wherein the supply flow control part (1520) is configured to synchronize the flow control of the mixed gas with respect to the inspiratory effort point (A) and expiratory effort point (B) synchronized in the breathing synchronization unit (1510).

3. The high flow respiratory therapy device (100) according to claim 1, wherein the supply flow control part (1520) is configured to set a basic flow (bias flow), which includes a flow level capable of ventilating the patient's nasal cavity with fresh air and the patient's maximum inhalation flow, is configured to supply the mixed gas of the bias flow when the patient's respiration starts, is configured to increase and supply the flow rate by adding a plus auxiliary flow (assist flow) to the bias flow at the inspiratory effort point (A), and is configured to reduce the flow rate to the bias flow at the expiratory effort point (B);

wherein the patient's maximum inhalation flow is determined to be three to four times the breathing capacity per minute clinically;
wherein the breathing capacity per minute is calculated as [one breath volume] × [number of breaths per minute].

**Patentansprüche**

1. Atemtherapievorrichtung mit hohem Durchfluss (100) durch Atemsynchronisation, umfassend

einen Teil zur Atemmusterüberwachung (1511), der darauf ausgelegt ist, Durchflussraten- und Druckänderungen eines einem Patienten zugeführten Mischgases zu überwachen, und darauf ausgelegt ist, die Atemmusterinformationen des Patienten zu sammeln;
einen Teil zur Inspirationsanstrengungspunktermittlung (1512), der darauf ausgelegt ist, einen Inspirationsanstrengungspunkt (A), an dem der Patient versucht, mit dem Einatmen zu beginnen, aus den Atemmusterinformationen des Patienten zu ermitteln;
einen Teil zur Exspirationsanstrengungspunktermittlung (1513), der darauf ausgelegt ist, einen Exspirationsanstrengungspunkt (B), an dem der Patient versucht, mit dem Ausatmen zu beginnen, aus den Atemmusterinformationen des Patienten zu ermitteln; und
einen Teil zur Versorgungsflusssteuerung (1520), der darauf ausgelegt ist, den Fluss des Mischgases zu erhöhen, wenn die Atmung des Patienten den Inspirationsanstrengungspunkt (A) erreicht, und darauf ausgelegt ist, den Fluss des Mischgases zu verringern, wenn die Atmung des Patienten den Exspirationsanstrengungspunkt (B) erreicht;
**dadurch gekennzeichnet, dass**:

der Teil zur Inspirationsanstrengungspunktermittlung (1512) darauf ausgelegt ist, einen maximalen Inspirationsanstrengungspunkt (C), an dem die Änderung der Einatmungsdurchflussrate maximiert ist, und einen Exspirationsanstrengungsendpunkt (E), an dem die Atmung vorübergehend stoppt, bevor nach dem Ausatmen wieder eingeatmet wird, aus den Atemmusterinformationen des Patienten zu extrahieren, und darauf ausgelegt ist, den Inspirationsanstrengungspunkt (A) zu ermitteln, an dem der Patient eine Inspirationsanstrengung zwischen dem extrahierten Exspirationsanstrengungsendpunkt (E) und dem maximalen Inspirationsanstrengungspunkt (C) beginnt, unter Bezugnahme auf den extrahierten Exspirationsanstrengungsendpunkt (E) und den maximalen Inspirationsanstrengungspunkt (C);
der Exspirationsanstrengungsendpunkt (E) aus einem Durchschnittswert extrahiert wird, der durch Addition der Atemflussrate des Patienten während eines Zyklus und Division durch die Anzahl der Zählungen ermittelt wird;
der Teil zur Inspirationsanstrengungspunktermittlung (1512) darauf ausgelegt ist, einen Skalarwert zwischen dem Exspirationsanstrengungsendpunkt (E) in einem ersten Zyklus und dem maximalen Inspirationsanstrengungspunkt (C) in einem zweiten Zyklus unter Bezugnahme auf mindestens zwei Zyklen zu berechnen und einen Wert innerhalb eines Bereichs von 10 % bis 30 % aus dem maximalen Inspirationsanstrengungspunkt (C) basierend auf dem berechneten Skalarwert als den Inspirationsanstrengungspunkt (A) zu ermitteln;
der Teil zur Exspirationsanstrengungspunktermittlung (1513) darauf ausgelegt ist, einen maximalen Exspirationsanstrengungspunkt (D), an dem die Änderung der Ausatmungsdurchflussrate des Patienten maximiert ist, und den Exspirationsanstrengungsendpunkt (E), an dem die Atmung vorübergehend stoppt, bevor nach dem Ausatmen wieder eingeatmet wird, aus den Atemmusterinformationen des Patienten zu extrahieren, und darauf ausgelegt ist, den Exspirationsanstrengungspunkt (B) zu ermitteln, an dem der Patient eine Exspirationsanstrengung zwischen dem extrahierten maximalen Exspirationsanstrengungspunkt (D) und dem Exspirationsanstrengungsendpunkt (E) unter Bezugnahme auf den extrahierten maximalen Exspirationsanstrengungspunkt (D) und den Exspirationsanstrengungsendpunkt (E) beginnt;
der Teil zur Exspirationsanstrengungspunktermittlung (1513) darauf ausgelegt ist, einen Skalarwert zwischen dem Exspirationsanstrengungsendpunkt (E) in einem ersten Zyklus und dem maximalen Exspirationsanstrengungspunkt (D) in einem zweiten Zyklus zu berechnen, der sich auf mindestens zwei Zyklen bezieht, und einen Wert innerhalb eines Bereichs von 10 % bis 30 % aus dem maximalen Exspirationsanstrengungspunkt (D) basierend auf dem berechneten Skalarwert als den Exspirationsanstrengungspunkt zu ermitteln.

2. Atemtherapievorrichtung mit hohem Durchfluss (100) nach Anspruch 1, die außerdem Folgendes umfasst:

eine Atmungssynchronisationseinheit (1510), die den Teil zur Atemmusterüberwachung (1511), den Teil zur Inspirationsanstrengungspunktermittlung (1512) und den Teil zur Exspirationsanstrengungspunktermittlung (1513) enthält und darauf ausgelegt ist, den Inspirationsanstrengungspunkt (A), der durch den Teil zur Inspirationsanstrengungspunkterfassung (1512) extrahiert wird, und den Exspirationsanstrengungspunkt (B), der durch den Teil der Exspirationsanstrengungspunktermittlung (1513) extrahiert wird, mit den Atemmusterinformationen des Patienten zu synchronisieren,
wobei der Teil zur Versorgungsflusssteuerung (1520) darauf ausgelegt ist, die Flusssteuerung des Mischgases im Verhältnis zu dem Inspirationsanstrengungspunkt (A) und dem Exspirationsanstrengungspunkt (B) zu synchronisieren, die in der Atmungssynchronisationseinheit (1510) synchronisiert sind.

3. Atemtherapievorrichtung mit hohem Durchfluss (100) nach Anspruch 1, wobei der Teil zur Versorgungsflusssteuerung (1520) darauf ausgelegt ist, einen Basisfluss (Bias Flow) einzustellen, der ein Flussniveau, das in der Lage ist, die Nasenhöhle des Patienten mit Frischluft zu belüften, und den maximalen Einatmungsfluss des Patienten umfasst, darauf ausgelegt ist, das Mischgas des Basisflusses zuzuführen, wenn die Atmung des Patienten beginnt, darauf ausgelegt ist, die Durchflussrate zu erhöhen und bereitzustellen, indem ein zusätzlicher Hilfsfluss (Unterstützungsfluss) zu dem Basisfluss an dem Inspirationsanstrengungspunkt (A) hinzugefügt wird, und darauf ausgelegt ist, die Durchflussrate zu dem Basisfluss an dem Exspirationsanstrengungspunkt (B) zu reduzieren;

wobei der maximale Einatmungsfluss des Patienten auf das Drei- bis Vierfache der klinischen Atemkapazität pro Minute festgelegt wird;
wobei die Atemkapazität pro Minute berechnet wird als [ein Atemzugvolumen] × [Anzahl der Atemzüge pro Minute].

**Revendications**

1. Dispositif de thérapie respiratoire à haut débit (100) par synchronisation respiratoire comprenant :

une partie de surveillance de profil respiratoire (1511), configurée pour surveiller les changements de débit et de pression d'un gaz mixte fourni à un patient et configurée pour collecter des informations de profil respiratoire du patient ;
une partie de détection de point d'effort inspiratoire (1512), configurée pour détecter un point d'effort inspiratoire (A), auquel le patient tente de commencer une inspiration, à partir des informations de profil respiratoire du patient ;
une partie de détection de point d'effort expiratoire (1513), configurée pour détecter un point d'effort expiratoire (B), auquel le patient tente de commencer une expiration, à partir des informations de profil respiratoire du patient ; et
une partie de régulation de débit d'alimentation (1520), configurée pour augmenter le débit du gaz mixte lorsque la respiration du patient atteint le point d'effort inspiratoire (A) et est configurée pour diminuer le débit du gaz mixte lorsque la respiration du patient atteint le point d'effort expiratoire (B) ;
**caractérisé en ce que** :

la partie de détection de point d'effort inspiratoire (1512) est configurée pour extraire un point d'effort inspiratoire maximal (C), auquel le changement de débit d'inspiration est maximisé, et un point de fin d'effort expiratoire (E), auquel la respiration s'arrête temporairement avant d'inspirer à nouveau après l'expiration, à partir des informations de profil respiratoire du patient, et est configurée pour détecter le point d'effort inspiratoire (A) auquel le patient commence un effort inspiratoire entre le point de fin d'effort expiratoire extrait (E) et le point d'effort inspiratoire maximal (C) en se référant au point de fin d'effort expiratoire extrait (E) et au point d'effort inspiratoire maximal (C) ;
le point de fin d'effort expiratoire (E) est extrait d'une valeur moyenne obtenue en additionnant le débit respiratoire du patient pendant un cycle et en le divisant par le nombre de comptes ;
la partie de détection de point d'effort inspiratoire (1512) est configurée pour calculer une valeur scalaire entre le point de fin d'effort expiratoire (E) dans un premier cycle et le point d'effort inspiratoire maximal (C) dans un deuxième cycle, en se référant à au moins deux cycles, et détecter une valeur dans une plage comprise entre 10 % et 30 % du point d'effort inspiratoire maximal (C) sur la base de la valeur scalaire calculée en tant que point d'effort inspiratoire (A) ;

la partie de détection de point d'effort expiratoire (1513) est configurée pour extraire un point d'effort expiratoire maximal (D), auquel le changement de débit expiratoire du patient est maximisé, et le point de fin d'effort expiratoire (E), auquel la respiration s'arrête temporairement avant d'inspirer à nouveau après l'expiration, à partir des informations de profil respiratoire du patient, et est configurée pour détecter le point d'effort expiratoire (B) auquel le patient commence un effort expiratoire entre le point d'effort expiratoire maximal extrait (D) et le point de fin d'effort expiratoire (E) en se référant au point d'effort expiratoire maximal extrait (D) et au point de fin d'effort expiratoire (E) ;

la partie de détection de point d'effort expiratoire (1513) est configurée pour calculer une valeur scalaire entre le point de fin d'effort expiratoire (E) dans un premier cycle et le point d'effort expiratoire maximal (D) dans un deuxième cycle, en se référant à au moins deux cycles, et détecter une valeur dans une plage comprise entre 10 % et 30 % du point d'effort expiratoire maximal (D) sur la base de la valeur scalaire calculée en tant que point d'effort expiratoire.

2. Dispositif de thérapie respiratoire à haut débit (100) selon la revendication 1, comprenant en outre :

une unité de synchronisation respiratoire (1510), qui inclut la partie de surveillance de profil respiratoire (1511), la partie de détection de point d'effort inspiratoire (1512) et la partie de détection de point d'effort expiratoire (1513), et qui est configurée pour synchroniser le point d'effort inspiratoire (A) extrait par la partie de détection de point d'effort inspiratoire (1512) et le point d'effort expiratoire (B) extrait par la partie de détection de point d'effort expiratoire (1513) avec les informations de profil respiratoire du patient,

dans lequel la partie de régulation de débit d'alimentation (1520) est configurée pour synchroniser la régulation de débit du gaz mixte par rapport au point d'effort inspiratoire (A) et au point d'effort expiratoire (B) synchronisés dans l'unité de synchronisation respiratoire (1510).

3. Dispositif de thérapie respiratoire à haut débit (100) selon la revendication 1, dans lequel la partie de régulation de débit d'alimentation (1520) est configurée pour régler un débit de base (débit de polarisation), qui comprend un niveau de débit capable de ventiler la cavité nasale du patient avec de l'air frais et le débit d'inspiration maximal du patient, est configurée pour fournir le gaz mixte du débit de polarisation lorsque la respiration du patient commence, est configurée pour augmenter et fournir le débit en ajoutant un débit auxiliaire positif (débit d'assistance) au débit de polarisation au point d'effort inspiratoire (A), et est configurée pour réduire le débit au débit de polarisation au point d'effort expiratoire (B) ;

dans lequel le débit d'inspiration maximal du patient est déterminé comme étant trois à quatre fois supérieur à la capacité respiratoire par minute, d'un point de vue clinique ;

dans lequel la capacité respiratoire par minute est calculée comme [un volume respiratoire] $\times$ [nombre de respirations par minute].

FIG. 1

FIG. 2

FIG. 3

_150A_

Flow rate change →
Pressure change →

Breathing pattern monitoring part — 1511

↓

Inspiratory effort point detection part — 1512

Expiratory effort point detection part — 1513

— 1510

↓

Supply flow control part according to inhalation/exhalation — 1520

FIG. 4

Flow rate (supply gas for patient)

2. Sustained Flow after increasing (Bias Flow + Assist flow)

3. Expiratory effort detection

4. Sustained Flow after decreasing (Bias Flow)

1. Inspiratory effort detection

Assist flow

Bias flow

Time

FIG. 5

FIG. 6

FIG. 7

```
                    ┌─────────┐
                    │  Start  │
                    └────┬────┘
                         │ ◄──────────────────────────┐
                         ▼                             │
   ┌─────────────────────────────────────────┐        │
   │ Perform respiratory synchronization     │   ── S100A
   │ process for detecting patient's         │        │
   │ breathing effort                        │        │
   └─────────────────┬───────────────────────┘        │
                     ▼                                 │
   ┌─────────────────────────────────────────┐        │
   │ Flow (bias flow/Assist flow)            │   ── S200A
   │ operation control according             │        │
   │ to inspiratory point and expiratory     │        │
   │ point                                   │        │
   └─────────────────┬───────────────────────┘        │
                     ▼                        ── S300A │ Command on
            ◇─────────────────────────────◇───────────┘
             Synchronization command/ End?
                     │ Enforce end
                     ▼
                ┌─────────┐
                │   End   │
                └─────────┘
```

FIG. 8

_150B_

```
                    ┌──────────────────────────────────────────────┐
                    │  ┌────────────────────────────────────────┐  │
 Flow rate change   │  │                                        │  │── 1511
 ─────────────────→ │  │  Breathing pattern monitoring part     │  │
 Pressure change    │  │                                        │  │
                    │  └────────────────────┬───────────────────┘  │
                    │                       ▼                       │
                    │  ┌────────────────────────────────────────┐  │── 1513
                    │  │  Expiratory effort point               │  │
                    │  │  detection part                        │  │
                    │  └────────────────────────────────────────┘  │── 1510
                    │                       │                       │
                    └───────────────────────┼───────────────────────┘
                                            ▼
                       ┌────────────────────────────────────────┐
                       │  Supply flow control part according     │
                       │  to exhalation                          │── 1520
                       └────────────────────────────────────────┘
```

FIG. 9

Flow rate
(supply gas for patient)

1. Expiratory effort
detection

3. Maintenance of flow increase
(Bias Flow + Relief Flow)

2. Flow rate increase
in proportion to decrease
of expiratory effort

Expiratory Assist flow (Relief Flow)

Basic flow (Bias Flow + Relief Flow)

Time

FIG. 10

Patient breathing flow

Time

Patient's inspiratory-expiratory effort

F. Expiratory
effort point

G. Expiratory effort end point

Time

FIG. 11

Start

Perform respiratory synchronization process
for detecting patient's breathing effort — *S100B*

Flow (bias flow/Assist flow)
operation control according
to expiratory point — *S200B*

Synchronization command/ End? — *S300B*

Command on

Enforce end

End

16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019217030 A1